**Europäisches Patentamt**

**European Patent Office**

**Office européen des brevets**

(19)

(11) Veröffentlichungsnummer : **0 324 712 B1**

(12)

# EUROPÄISCHE PATENTSCHRIFT

(45) Veröffentlichungstag der Patentschrift :
07.04.93 Patentblatt 93/14

(51) Int. Cl.⁵ : **C07K 7/10,** C12N 15/00,
C12P 21/02, A61K 37/64

(21) Anmeldenummer : **89710003.8**

(22) Anmeldetag : **11.01.89**

(54) **Hirudin-Derivat.**

(30) Priorität : **13.01.88 HU 134185
23.02.88 DE 3805540**

(43) Veröffentlichungstag der Anmeldung :
**19.07.89 Patentblatt 89/29**

(45) Bekanntmachung des Hinweises auf die
Patenterteilung :
**07.04.93 Patentblatt 93/14**

(84) Benannte Vertragsstaaten :
**AT BE CH DE ES FR GB GR IT LI LU NL SE**

(56) Entgegenhaltungen :
**EP-A- 0 158 986
EP-A- 0 209 061**

(73) Patentinhaber : **HOECHST
AKTIENGESELLSCHAFT
Postfach 80 03 20
W-6230 Frankfurt am Main 80 (DE)**

(72) Erfinder : **Crause, Peter, Dr.
Schopenhauerstrasse 31
W-6050 Offenbach (DE)**
Erfinder : **Habermann, Paul, Dr.
Rossertstrasse 35
W-6239 Eppstein Taunus (DE)**
Erfinder : **Tripier, Dominique, Dr.
Am Kirschgarten 18
W-6239 Eppstein Taunus (DE)**

EP 0 324 712 B1

**Beschreibung**

Aus der europäischen Patentanmeldung mit der Veröffentlichungsnummer (EP-A) 0 171 024 sind Derivate des Hirudin und ihre gentechnische Herstellung bekannt.

Es wurde nun gefunden, daß das Hirudin-Derivat der Aminosäuresequenz

```
        0     1                                              10
        Leu- Thr- Tyr- Thr- Asp- Cys- Thr- Glu- Ser- Gly- Gln- Asn- Leu- Cys-
                                            20
        Leu- Cys- Glu- Gly- Ser- Asn- Val- Cys- Gly- Gln- Gly- Asn- Lys- Cys-
                 30                                                    40
        Ile- Leu- Gly- Ser- Asp- Gly- Glu- Lys- Asn- Gln- Cys- Val- Thr- Gly-
                                            50
        Glu- Gly- Thr- Pro- Lys- Pro- Gln- Ser- His- Asn- Asp- Gly- Asp- Phe-
                              60
        Glu- Glu- Ile- Pro- Glu- Glu- Tyr- Leu- Gln
```

eine Reihe von Vorteilen aufweist. In dieser Sequenz wurde die Numerierung gemäß EP-A 0 171 024 beibehalten.

Das Hirudin und seine Derivate weisen unterschiedliche biologische Aktivität auf, was auf eine unterschiedliche Affinität zum Thrombin und/oder eine unterschiedliche Stabilität zurückgeführt werden kann. Das erfindungsgemäße Hirudin-Derivat zeichnet sich überraschenderweise durch eine besondere Aktivität aus.

Weiterhin wurde gefunden, daß das erfindungsgemäße Hirudin-Derivat besonders vorteilhaft in Hefen exprimiert wird. Wie Vergleichsversuche zeigten, erfolgt eine Expression von analogen Hirudin-Derivaten, die N-terminal mit Thr-Tyr oder Ile-Tyr beginnen, nur mit niedrigen Ausbeuten.

Die Expression aus Hefezellen ist nicht nur deshalb vorteilhaft, weil das Hirudin-Derivat sekretiert wird, sondern vor allem deshalb, weil es praktisch quantitativ in richtig gefalteter Form vorliegt und hohe Aktivität zeigt.

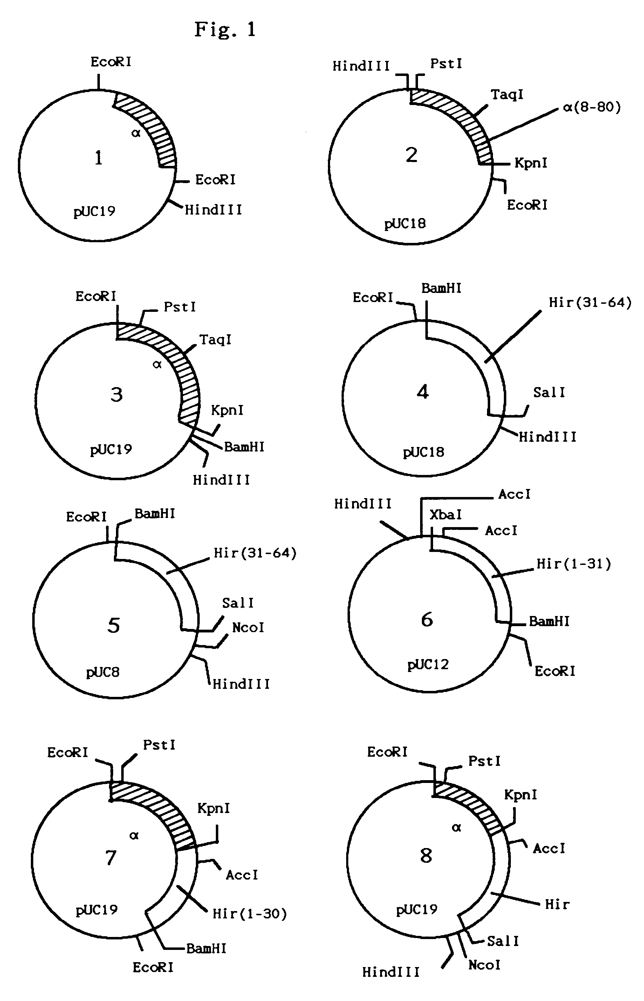

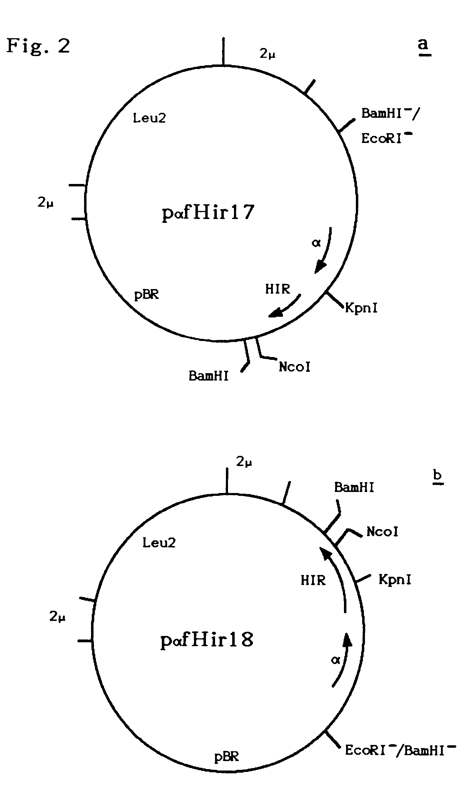

Die Figur 1 zeigt Klonierungsvektoren zur Gewinnung einer Genstruktur, die für das Hefe-MFα-Vorläuferprotein und das erfindungsgemäße Hirudinderivat codiert. Die Figur 2 zeigt einen Hefe-Expressionsvektor mit dieser Genstruktur.

Die Herstellung des erfindungsgemäßen Hirudin-Derivats kann selbstverständlich auch nach anderen Methoden erfolgen, beispielsweise durch Expression in Bakterien oder in höheren eukaryotischen Zellen wie Insektenzellen oder tierischen Zellen. Bevorzugt wird jedoch die Expression aus Hefesystemen, beispielsweise unter Verwendung der Hefe-Arten, wie sie in der EP-A 0 248 227 aufgeführt sind, z. B. Pichia pastoris, Hansenula polymorphis, Schizosaccharomyces pombe oder bevorzugt Saccharomyces cerevisiae.

Vektoren für die Expression in Hefen sind in großer Zahl bekannt, beispielsweise aus EP-A 0 060 057, 0 088 632, 0 116 201, 0 121 884, 0 123 544 und 0 195 691. Die Herstellung des erfindungsgemäßen Hirudin-Derivats wird im folgenden anhand des Hefe-α-Faktorsystems beschrieben, was jedoch nur als beispielhaft zu verstehen ist, da in an sich bekannter Weise auch andere Expressionssysteme eingesetzt werden können.

Die Struktur des Hefe-Pheromongens MFα ist bekannt aus Kurjan und Hershkovitz, Cell 30 (1982) 933-943, wo auch die Möglichkeit der Expression anderer Gene und die Sekretion der Genprodukte diskutiert wird. Diesbezüglich kann auch auf Brake et al., Proc. Natl. Acad. Sci. USA 81 (1984, 4642-4646, verwiesen werden.

Als Hefevektoren werden vorteilhaft sogenannte "shuttle"-Vektoren verwendet, die einen bakteriellen Plasmid- und einen Hefeplasmid-Replikationsursprung sowie Gene zur Selektion in beiden Wirtssystemen aufweisen. Ferner enthalten solche Vektoren die zur Expression fremder Gene notwendigen Promotorsequenzen und gegebenenfalls zur Verbesserung der Ausbeute eine Terminatorsequenz, so daß das heterologe Gen - zweckmäßig fusioniert an sekretorische Signale - zwischen Promotor und Terminator angeordnet ist.

Die Erfindung wird durch die folgenden Beispiele näher erläutert. Prozentangaben beziehen sich auf das Gewicht.

**Beispiel 1: Konstruktion des Expressionsvektors**

Zunächst wird die DNA-Sequenz I (Tabelle 1) nach dem Phosphitverfahren synthetisiert. Diese DNA-Sequenz codiert für die Aminosäuren 49 bis 80 des MFα-Vorläuferproteins und entspricht im wesentlichen der natürlichen DNA-Sequenz.

Die DNA-Sequenz I wird zunächst als Sonde zur Isolierung des Gens für den α-Faktor verwendet und hierzu mit $^{32}$P markiert. Mit Hilfe dieser Sonde wird aus einer genomischen λgt11-Hefegenbank (wie sie inzwischen handelsüblich und z. B. bei Clontech Laboratories Inc., 4055 Fabian Way, Palo Alto, CA94303 erhältlich sind) das Gen isoliert. Dazu werden λgt11-Phagen, die das α-Faktorgen tragen, in einem Plaque-Hybridisierungsexperiment identifiziert. Phagen aus als positiv identifizierten Plaques werden isoliert, vermehrt und die DNA gewonnen. Diese wird mit EcoRI gespalten und auf einem 0,8%-igen Agarosegel analysiert. Nach einem "Southern transfer"-Experiment wird die Membran gegen die $^{32}$P-markierte DNA-Sequenz I hybridisiert. Phagen-DNA, die ein ca. 1,75 kb-Fragment aufweist, das gegen die DNA-Sequenz I hybridisiert, wird erneut mit dem Enzym gespalten und das entsprechende Fragment isoliert. Der Vektor pUC 19 wird mit EcoRI geöffnet und mit dem 1,75 kb-Fragment mit T4-Ligase umgesetzt. Man erhält den Klonierungsvektor 1.

In der Tabelle 2 sind die Klonierungsvektoren aufgeführt, die alle auf Basis eines pUC-Plasmids konstruiert wurden. Die Tabelle zeigt hierbei nur die Polylinker-Region dieser Vektoren in der üblichen 5′-3′-Richtung, wobei die MFα-Sequenzen durch punktierte und die Hirudin-Sequenzen durch gestrichelte Linien angedeutet sind. Durchgezogene Linien bedeuten pUC- bzw. Linker-Sequenzen. Die Figur 1 zeigt diese Klonierungsvektoren schematisch und nicht maßstabsgetreu.

Mit dem Ligationsgemisch wird der Stamm E. coli 79/02 transformiert. Weiße Kolonien werden isoliert, hieraus die Plasmid-DNA gewonnen und Plasmide, die das 1,75 kb-EcoRI-Fragment enthalten, identifiziert.

Die natürliche DNA-Sequenz des Vorläuferproteins für MFα enthält im Bereich der Codons für die Aminosäuren 8 bis 10 eine PstI-Schnittstelle und im Bereich der Codons für die Aminosäuren 48/49 eine TaqI-Schnittstelle. Aus der isolierten Plasmid-DNA wird nun durch Umsetzung mit PstI und TaqI das Fragment isoliert, das für die Aminosäuren 9 bis 48 der MFα-Vorläufersequenz codiert. Der Vektor pUC18 wird mit PstI und KpnI geöffnet und mit dem PstI-TaqI-Fragment sowie mit der synthetischen DNA-Sequenz I mit Hilfe von T4-Ligase umgesetzt. Mit dem Ligationsgemisch wird E. coli 79/02 transformiert. Das Transformationsgemisch wird auf IPTG-Xgal-Ap-Platten ausplattiert. Weiße Kolonien werden isoliert und die Plasmid-DNA dieser Klone durch Restriktionsanalyse charakterisiert. Man erhält so den Klonierungsvektor 2, der für die Aminosäuren 8 bis 80 der MFα-Vorläufersequenz codiert.

Aus dem Klonierungsvektor 2 wird durch Umsetzung mit PstI und KpnI die genannte codierende Sequenz ausgeschnitten und in die im folgenden beschriebene Ligierung eingebracht. Hierzu wird der Klonierungsvektor 1 mit EcoRI und partial mit PstI umgesetzt und das die Codierungssequenz für die ersten 8 Aminosäuren der MFα-Vorläufersequenz umfassende Fragment isoliert. Weiterhin wird der Vektor pUC19 mit EcoRI und KpnI geöffnet und mit den beiden beschriebenen Fragmenten ligiert, wobei der Klonierungsvektor 3 entsteht. Dieser codiert für die gesamte Vorläufersequenz des MFα bis zur Aminosäure 80.

Als Ausgangsmaterial für den größten Teil der Hirudin-Sequenz dient das in der EP-A 0 171 024 als "DNA-Sequenz I" wiedergegebene synthetische Gen, das in der vorliegenden Tabelle 1 als DNA-Sequenz IV aufgeführt ist. In dieser Sequenz sind die Restriktionsenzym-Schnittstellen durch Unterstreichung hervorgehoben: Im Bereich der Aminosäuren 1 bis 3 schneidet AccI, im Bereich der Aminosäuren 30/31 BamHI und, beginnend mit dem letzten Stop-Codon, SacI. Am 5′-Ende des Gens findet sich die überhängende Sequenz für XbaI und am 3′-Ende die überhängende Sequenz für SalI.

Dieses synthetische Gen wurde in zwei Teilen subkloniert (Figuren 1 und 2 in der EP-A 0 171 024). Diese Subklonierungsvektoren sind in der Tabelle 2 unter Nr. 4 (entsprechend Figur 2 von EP-A 0 171 024) bzw. 6 (entsprechend der Figur 1 von EP-A 0 171 024) wiedergegeben.

Der Klonierungsvektor 4 wird mit HincII und HindIII geöffnet und die linearisierte DNA wird mit der DNA-Sequenz II (Tabelle 1) ligiert. In dem so erhaltenen Klonierungsvektor 5 ist an der stumpfendig ligierten Stelle eine NcoI-Schnittstelle gebildet worden.

Aus dem Klonierungsvektor 6 wird das für die Hirudin-Teilsequenz codierende Fragment durch Totalverdauung mit BamHI und AccI herausgeschnitten. Dieses Fragment wird dann mit dem Klonierungsvektor 3, der mit BamHI und KpnI geöffnet wurde, sowie mit der DNA-Sequenz III (Tabelle 1) ligiert. In der DNA-Sequenz III sind die letzten drei Codons in der gleichen Weise numeriert wie in der DNA-Sequenz IV (Tabelle 1). Man erhält so den Klonierungsvektor 7, der für die ersten 80 Aminosäuren der Vorläufersequenz von MFα und die ersten 30 Aminosäuren des erfindungsgemäßen Hirudin-Derivats codiert, wie durch DNA-Sequenzanalyse bestätigt wurde.

Aus dem Klonierungsvektor 5 wird mit BamHI und HindIII das Fragment ausgeschnitten, das für die Aminosäuren 31 bis 64 von Hirudin codiert. Dieses Fragment wird in den mit den gleichen Enzymen geöffneten Klonierungsvektor 7 ligiert, wobei der Klonierungsvektor 8 erhalten wird, der für die ersten 80 Aminosäuren der MFα-Vorläufersequenz und die gesamte Sequenz des erfindungsgemäßen Hirudin-Derivats codiert. Die Struktur dieses Plasmids wird durch Restriktionsanalyse bestätigt.

Das Plasmid Yep13 (Broach et al., Gene 8 (1979) 121) wird mit BamHI geöffnet und die überstehenden Enden mit Klenow-Polymerase aufgefüllt. Die DNA wird mit Ethanol gefällt und mit alkalischer Rinderphosphatase behandelt.

Aus dem Klonierungsvektor 8 (Tabelle 2) wird mit NcoI und EcoRI das für das Hirudin-Derivat und die Vorläufersequenz des MFα codierende Fragment ausgeschnitten und die überstehenden Enden wie beschrieben aufgefüllt.

Die beiden stumpfendigen DNA-Sequenzen werden miteinander ligiert, wobei die Plasmide pαfHir17 und pαfHir18 (Figur 2) entstehen. Diese beiden Plasmide unterscheiden sich nur in der Orientierung des insertierten Fragmentes.

Wie in der EP-A 0 171 024 beschrieben, kann hinter die insertierte Sequenz ein Terminator eingesetzt werden (Figuren 4 bis 6 der EP-A 0 171 024). Hierfür eignen sich die NcoI- und/oder die BamHI-Schnittstellen.

Nach Amplifikation der Plasmid-DNA in E. coli MM294 wird das Plasmid pαfHir17 in die Leucin-bedürftigen Hefestämme Y79 (α,trp1-1,leu2-1) (Cantrell et al., Proc. Acad. Natl. Sci. USA 82 (1985) 6250) und DM6-6(α/α leu2-3,112::ura3$^+$/leu2::lys2$^+$, trp1$^-$/trp1$^-$, his3-11, 15/his3-11, 15, ura3$^-$/ura3$^-$, lys2$^-$/lys2$^-$, arg4-17/arg4$^+$, ade1$^-$/ade1$^+$) (Maya Hanna, Dept. Mol. Biol. Massachusetts General Hospital, Boston, USA) nach der Lithium-Methode von Ito, H. et al., J. Bacteriol., 153 (1983) 163 transformiert. Kolonien, die auf selektivem Medium ohne Leucin-Zusatz wachsen können, werden isoliert und vereinzelt. Hefe-Minimalmedium wird mit den einzelnen Kolonien beimpft und 24 Stunden bei 28°C inkubiert. Die Zellen werden abzentrifugiert und der Überstand in einem Thrombin-Hemmtest auf Hirudinaktivität überprüft. Aus Hefeklonen, deren Überstand Hirudin-Aktivität zeigt, wird die Plasmid-DNA reisoliert und durch Restriktionsanalyse charakterisiert. Die transformierten Hefestämme werden für die folgenden Expressionsversuche eingesetzt.

## Beispiel 2: Expression

10 ml Hefevollmedium wird mit Zellen, die aus einer frischen Übernachtkultur eines nach Beispiel 1 erhaltenen Stammes aus selektivem Medium entnommen wurden, so beimpft, daß eine optische Dichte $OD_{600}$ = 0,1 erreicht wird. Die Kultur wird 8 Stunden bei 28°C geschüttelt, worauf 90 ml frisches Medium zugesetzt werden. Anschließend wird die Kultur für weitere 20 Stunden geschüttelt. Die Zellen werden abzentrifugiert und die Hirudinaktivität im Überstand bestimmt.

## Beispiel 3: Aufarbeitung

Nach Beispiel 2 erhaltener Überstand wird auf pH 3 bis 5 angesäuert und auf eine mit 0,1 M Essigsäure äquilibrierte Adsorptionssäule mit einem porösen Adsorberharz aus einem Copolymer von Styrol und Divinylbenzol (®DIAION HP 20) gegeben. Nach Waschen mit Tris · HCl (pH 8,5) und 50 mM Essigsäure erfolgt die Elution mit 30 %igem Isopropanol. Die das Hirudin-Derivat enthaltenden Fraktionen werden vereinigt und über eine Q-SEPHAROSE®-Säule gereinigt, die mit 20 mM Piperazin · HCl (pH 6) äquilibriert wurde. Die Elution erfolgt hierbei über einen 0 - 0,25 M NaCl-Gradienten. Die das Hirudin-Derivat enthaltenden Fraktionen werden erneut vereinigt und durch HPLC über eine C18-"Reversed Phase"-Chromatographiesäule gereinigt. Das so erhaltene Reinprodukt wird anschließend einer automatisierten Proteinsequenzanalyse unterzogen.

## Beispiel 4: Vergleichsbeispiel

Verfährt man gemäß Beispiel 1, setzt jedoch an Stelle der DNA-Sequenz III (Tabelle 1) die folgenden Sequenzen ein, so kann man im Überstand der Hefekultur nur eine minimale Hirudin-Aktivität nachweisen.

|      |     |       | (Pro) | Leu | Asp | Lys | Arg | Thr<br>1 | (Tyr)<br>2 |
|------|-----|-------|-------|-----|-----|-----|-----|-----|-------|
|      | 5'  |       | CT    | TTG | GAT | AAA | AGA | ACG | T     |
| IIIa | 3'  | CAT   | GGA   | AAC | CTA | TTT | TCT | TGC | ATA   |
|      |     | (KpnI) |       |     |     |     |     | (AccI) |    |

```
                                                                  1       2
                           (Pro)   Leu    Asp    Lys    Arg    Ile   (Tyr)
               5'                  CT     TTG    GAT    AAA    AGA    ATA    T
      IIb      3'   CAT    GGA    AAC    CTA    TTT    TCT    TAT    ATA
               (KpnI)
```

Bei Verwendung der DNA-Sequenz IIIb enthalten die den Klonierungsvektoren 7 und 8 (Tabelle 2) entsprechenden Vektoren nicht die AccI-Schnittstelle.

## Tabelle 1: DNA-Sequenzen

```
                          50                           55
      I.   5'      C GAT GTT GCT GTT TTG CCA TTC TCC
           3'        TA CAA CGA CAA AAC GGT AAG AGG
           (TaqI)
                              60                           65
                 AAC AGT ACT AAT AAC GGT TTA TTG TTC
                 TTG TCA TGA TTA TTG CCA AAT AAC AAG


                              70
                 ATT AAT ACT ACT ATT GCT AGC ATT GCT
                 TAA TTA TGA TGA TAA CGA TCG TAA CGA


                 75                    80
                 GCT AAA GAA GAA GGG GTA C      3'
                 CGA TTT CTT CTT CCC            5'
                                     (KpnI)



      II.  5'     CATGGA            3'
           3'     GTACCTTCGA        5'
                       (HindIII)



                      (Pro) Leu   Asp   Lys   Arg   Leu   Thr  (Tyr)
      III. 5'           CT  TTG   GAT   AAA   AGA   CTT   ACG   T       3'
           3' CAT  GGA  AAC  CTA   TTT   TCT   GAA   TGC   ATA          5'
              (KpnI)                                       (AccI)
```

DNA-Sequenz IV

| Triplett Nr. | | | | | | 0 | 1 | 2 | 3 | 4 | 5 |
|---|---|---|---|---|---|---|---|---|---|---|---|
| Aminosäure | | | | | | Met | Thr | Tyr | Thr | Asp | Cys |
| Nucleotid Nr. | | | 1 | | | | 10 | | | 20 | |
| Cod. Strang | | 5' | CT | AGA | ATG | ACG | TAT | ACT | GAC | TGC |
| nicht cod. Strang | | 3' | T | TAC | TGC | ATA | TGA | CTG | ACG |

| 6 | 7 | 8 | 9 | 10 | 11 | 12 | 13 | 14 | 15 |
|---|---|---|---|---|---|---|---|---|---|
| Thr | Glu | Ser | Gly | Gln | Asn | Leu | Cys | Leu | Cys |
| | | 30 | | | 40 | | | 50 | |
| ACT | GAA | TCT | GGT | CAG | AAC | CTG | TGC | CTG | TGC |
| TGA | CTT | AGA | CCA | GTC | TTG | GAC | ACG | GAC | ACG |

| 16 | 17 | 18 | 19 | 20 | 21 | 22 | 23 | 24 | 25 |
|---|---|---|---|---|---|---|---|---|---|
| Glu | Gly | Ser | Asn | Val | Cys | Gly | Gln | Gly | Asn |
| | | 60 | | | 70 | | | 80 | |
| GAA | GGA | TCT | AAC | GTT | TGC | GGC | CAG | GGT | AAC |
| CTT | CCT | AGA | TTG | CAA | ACG | CCG | GTC | CCA | TTG |

| 26 | 27 | 28 | 29 | 30 | 31 | 32 | 33 | 34 | 35 |
|---|---|---|---|---|---|---|---|---|---|
| Lys | Cys | Ile | Leu | Gly | Ser | Asp | Gly | Glu | Lys |
| | | 90 | | | 100 | | | 110 | |
| AAA | TGC | ATC | CTT | GGA | TCC | GAC | GGT | GAA | AAG |
| TTT | ACG | TAG | GAA | CCT | AGG | CTG | CCA | CTT | TTC |

| 36 | 37 | 38 | 39 | 40 | 41 | 42 | 43 | 44 | 45 |
|---|---|---|---|---|---|---|---|---|---|
| Asn | Gln | Cys | Val | Thr | Gly | Glu | Gly | Thr | Pro |
| | | 120 | | | 130 | | | 140 | |
| AAC | CAG | TGC | GTT | ACT | GGC | GAA | GGT | ACC | CCG |
| TTG | GTC | ACG | CAA | TGA | CCG | CTT | CCA | TGG | GGC |

| 46 | 47 | 48 | 49 | 50 | 51 | 52 | 53 | 54 | 55 |
|---|---|---|---|---|---|---|---|---|---|
| Lys | Pro | Gln | Ser | His | Asn | Asp | Gly | Asp | Phe |
| | | 150 | | | 160 | | | 170 | |
| AAA | CCG | CAG | TCT | CAT | AAC | GAC | GGC | GAC | TTC |
| TTT | GGC | GTC | AGA | GTA | TTG | CTG | CCG | CTG | AAG |

| 56 | 57 | 58 | 59 | 60 | 61 | 62 | 63 | 64 | |
|---|---|---|---|---|---|---|---|---|---|
| Glu | Glu | Ile | Pro | Glu | Glu | Tyr | Leu | Gln | Stp |
| | | 180 | | | 190 | | | 200 | |
| GAA | GAG | ATC | CCT | GAG | GAA | TAC | CTT | CAG | TAA |
| CTT | CTC | TAG | GGA | CTC | CTT | ATG | GAA | GTC | ATT |

| Stp | | | | | | | | | |
|---|---|---|---|---|---|---|---|---|---|
| | | 210 | | | | | | | |
| TAG | AGC | TCG | | | 3' | | | | |
| ATC | TCG | AGC | AGC | T | 5' | | | | |

## Tabelle 2: Klonierungsvektoren

| Nr. | pUC | |
|-----|-----|---|
| 1 | 19 | -E···(1,75 kb α-Fragment)···E- |
| 2 | 18 | -K···(α-80-49)···T···(α-48-8)···P- |
| 3 | 19 | -B-K···(α-80-49)···T···(α-48-8)···P···E- |
| 4 | 8 | -B---(Hir31-64)-S-Hc-Hd- |
| 5 | 8 | -B---(Hir31-64)-S-N-Hd- |
| 6 | 12 | -B---(Hir30-3)---A---X-A- |
| 7 | 19 | -Hd-B---(Hir30-3)---A---K···(α-80-8)···P···E- |
| 8 | 19 | -Hd-N-S---(Hir64-3)---A---K···(α-80-8)···P···E- |

··· MFα-Sequenzen        ---Hirudin-Sequenzen

Abkürzungen für Restriktionsenzyme

A = AccI
B = BamHI
E = EcoRI
Hc = HincII
Hd = HindIII
K = KpnI
N = NcoI
P = PstI
S = SalI
T = TaqI
X = XbaI

**Patentansprüche**

**Patentansprüche für folgende Vertragsstaaten : AT, BE, CH, DE, FR, GB, IT, LI, LU, NL, SE**

1.  Hirudin-Derivat der Aminosäuresequenz

0   1                                                10
Leu-Thr-Tyr-Thr-Asp-Cys-Thr-Glu-Ser-Gly-Gln-Asn-Leu-Cys-
                          20
Leu-Cys-Glu-Gly-Ser-Asn-Val-Cys-Gly-Gln-Gly-Asn-Lys-Cys-
        30                                          40
Ile-Leu-Gly-Ser-Asp-Gly-Glu-Lys-Asn-Gln-Cys-Val-Thr-Gly-
                          50
Glu-Gly-Thr-Pro-Lys-Pro-Gln-Ser-His-Asn-Asp-Gly-Asp-Phe-
                          60
Glu-Glu-Ile-Pro-Glu-Glu-Tyr-Leu-Gln

7

2. DNA, codierend für das Polypeptid mit der Aminosäure-Sequenz nach Anspruch 1.

3. Vektoren, enthaltend eine DNA-Sequenz nach Anspruch 2.

4. Verfahren zur Herstellung eines Polypeptids nach Anspruch 1, dadurch gekennzeichnet, daß man eine DNA nach Anspruch 2 in einer Wirtszelle exprimiert.

5. Verfahren nach Anspruch 4, dadurch gekennzeichnet, daß die Wirtszelle eine Hefezelle ist.

6. Arzneimittel, gekennzeichnet durch einen Gehalt an dem Polypeptid nach Anspruch 1.

**Patentansprüche für folgenden Vertragsstaat : GR**

1. DNA, codierend für ein Hirudin-Derivat der Aminosäuresequenz

```
 0   1                                    10
Leu-Thr-Tyr-Thr-Asp-Cys-Thr-Glu-Ser-Gly-Gln-Asn-Leu-Cys-
                             20
Leu-Cys-Glu-Gly-Ser-Asn-Val-Cys-Gly-Gln-Gly-Asn-Lys-Cys-
        30                                   40
Ile-Leu-Gly-Ser-Asp-Gly-Glu-Lys-Asn-Gln-Cys-Val-Thr-Gly-
                             50
Glu-Gly-Thr-Pro-Lys-Pro-Gln-Ser-His-Asn-Asp-Gly-Asp-Phe-
                      60
Glu-Glu-Ile-Pro-Glu-Glu-Tyr-Leu-Gln.
```

2. Vektoren, enthaltend eine DNA-Sequenz nach Anspruch 1.

3. Verfahren zur Herstellung eines Polypeptids mit der in Anspruch 1 genannten Aminosäuresequenz, dadurch gekennzeichnet, daß man eine DNA nach Anspruch 1 in einer Wirtszelle exprimiert.

4. Verfahren nach Anspruch 3, dadurch gekennzeichnet, daß die Wirtszelle eine Hefezelle ist.

**Patentansprüche für folgenden Vertragsstaat : ES**

1. Verfahren zur Herstellung eines Hirudin-Derivats der Aminosäuresequenz

```
 0   1                                    10
Leu-Thr-Tyr-Thr-Asp-Cys-Thr-Glu-Ser-Gly-Gln-Asn-Leu-Cys-
                             20
Leu-Cys-Glu-Gly-Ser-Asn-Val-Cys-Gly-Gln-Gly-Asn-Lys-Cys-
        30                                   40
Ile-Leu-Gly-Ser-Asp-Gly-Glu-Lys-Asn-Gln-Cys-Val-Thr-Gly-
                             50
Glu-Gly-Thr-Pro-Lys-Pro-Gln-Ser-His-Asn-Asp-Gly-Asp-Phe-
                      60
Glu-Glu-Ile-Pro-Glu-Glu-Tyr-Leu-Gln,
```

dadurch gekennzeichnet, daß man eine DNA, die für dieses Polypeptid codiert, in einer Wirtszelle exprimiert.

2.  Verfahren nach Anspruch 1, dadurch gekennzeichnet, daß die Wirtszelle eine Hefezelle ist.


**Claims**

**Claims for the following Contracting States : AT, BE, CH, DE, FR, GB, IT, LI, LU, NL, SE**

1.  A hirudin derivative with the amino acid sequence


```
      0     1                                          10
     Leu-Thr-Tyr-Thr-Asp-Cys-Thr-Glu-Ser-Gly-Gln-Asn-Leu-
                                 20
     Cys-Leu-Cys-Glu-Gly-Ser-Asn-Val-Cys-Gly-Gln-Gly-Asn-
                                 30
     Lys-Cys-Ile-Leu-Gly-Ser-Asp-Gly-Glu-Lys-Asn-Gln-Cys-
        40                                          50
     Val-Thr-Gly-Glu-Gly-Thr-Pro-Lys-Pro-Gln-Ser-His-Asn-
                                          60
     Asp-Gly-Asp-Phe-Glu-Glu-Ile-Pro-Glu-Glu-Tyr-Leu-Gln.
```

2.  DNA coding for the polypeptide having the amino acid sequence as claimed in claim 1.

3.  Vectors containing a DNA sequence as claimed in claim 2.

4.  A process for the preparation of a polypeptide as claimed in claim 1, which comprises expression of a DNA as claimed in claim 2 in a host cell.

5.  The process as claimed in claim 4, wherein the host cell is a yeast cell.

6.  A pharmaceutical containing a polypeptide as claimed in claim 1.

**Claims for the following Contracting State : GR**

1.  DNA coding for a hirudin derivative with the amino acid sequence

```
       0    1                                       10
     Leu-Thr-Tyr-Thr-Asp-Cys-Thr-Glu-Ser-Gly-Gln-Asn-Leu-
                                     20
     Cys-Leu-Cys-Glu-Gly-Ser-Asn-Val-Cys-Gly-Gln-Gly-Asn-
                       30
     Lys-Cys-Ile-Leu-Gly-Ser-Asp-Gly-Glu-Lys-Asn-Gln-Cys-
             40                                      50
     Val-Thr-Gly-Glu-Gly-Thr-Pro-Lys-Pro-Gln-Ser-His-Asn-
                                     60
     Asp-Gly-Asp-Phe-Glu-Glu-Ile-Pro-Glu-Glu-Tyr-Leu-Gln.
```

2.  Vectors containing a DNA sequence as claimed in claim 1.

3.  A process for the preparation of a polypeptide with the amino acid sequence mentioned in claim 1, which comprises expression of a DNA as claimed in claim 1 in a host cell.

4.  The process as claimed in claim 3, wherein the host cell is a yeast cell.

**Claims for the following Contracting State : ES**

1.  A process for the preparation of a hirudin derivative with the amino acid sequence

```
       0    1                                       10
     Leu-Thr-Tyr-Thr-Asp-Cys-Thr-Glu-Ser-Gly-Gln-Asn-Leu-
                                     20
     Cys-Leu-Cys-Glu-Gly-Ser-Asn-Val-Cys-Gly-Gln-Gly-Asn-
                       30
     Lys-Cys-Ile-Leu-Gly-Ser-Asp-Gly-Glu-Lys-Asn-Gln-Cys-
             40                                      50
     Val-Thr-Gly-Glu-Gly-Thr-Pro-Lys-Pro-Gln-Ser-His-Asn-
                                     60
     Asp-Gly-Asp-Phe-Glu-Glu-Ile-Pro-Glu-Glu-Tyr-Leu-Gln,
```

which comprises expression of a DNA which codes for this polypeptide in a host cell.

2.  The process as claimed in claim 1, wherein the host cell is a yeast cell.

**Revendications**

**Revendications pour les Etats contractants suivants : AT, BE, CH, DE, FR, GB, IT, LI, LU, NL, SE**

1.  Dérivé de l'hirudine, de séquence d'aminoacides

```
    0     1                                          10
  Leu-Thr-Tyr-Thr-Asp-Cys-Thr-Glu-Ser-Gly-Gln-Asn-Leu-Cys-
                              20
  Leu-Cys-Glu-Gly-Ser-Asn-Val-Cys-Gly-Gln-Gly-Asn-Lys-Cys-
            30                                      40
  Ile-Leu-Gly-Ser-Asp-Gly-Glu-Lys-Asn-Gln-Cys-Val-Thr-Gly-
                              50
  Glu-Gly-Thr-Pro-Lys-Pro-Gln-Ser-His-Asn-Asp-Gly-Asp-Phe-
                    60
  Glu-Glu-Ile-Pro-Glu-Glu-Tyr-Leu-Gln.
```

**2.** ADN, codant pour le polypeptide ayant la séquence d'aminoacides selon la revendication 1.

**3.** Vecteurs, contenant une séquence d'ADN selon la revendication 2.

**4.** Procédé pour préparer un polypeptide selon la revendication 1, caractérisé en ce qu'on exprime un ADN selon la revendication 2, dans une cellule hôte.

**5.** Procédé selon la revendication 4, caractérisé en ce que la cellule hôte est une cellule de levure.

**6.** Médicament, caractérisé par une teneur en polypeptide selon la revendication 1.

**Revendications pour l'Etat contractant suivant : GR**

**1.** ADN, codant pour un dérivé de l'hirudine, de séquence d'aminoacides

```
    0     1                                          10
  Leu-Thr-Tyr-Thr-Asp-Cys-Thr-Glu-Ser-Gly-Gln-Asn-Leu-Cys-
                              20
  Leu-Cys-Glu-Gly-Ser-Asn-Val-Cys-Gly-Gln-Gly-Asn-Lys-Cys-
            30                                      40
  Ile-Leu-Gly-Ser-Asp-Gly-Glu-Lys-Asn-Gln-Cys-Val-Thr-Gly-
                              50
  Glu-Gly-Thr-Pro-Lys-Pro-Gln-Ser-His-Asn-Asp-Gly-Asp-Phe-
                    60
  Glu-Glu-Ile-Pro-Glu-Glu-Tyr-Leu-Gln.
```

**2.** Vecteurs, contenant une séquence d'ADN selon la revendication 1.

3. Procédé pour préparer un polypeptide ayant la séquence d'aminoacides citée dans la revendication 1, caractérisé en ce qu'on exprime un ADN selon la revendication 1, dans une cellule hôte.

4. Procédé selon la revendication 3, caractérisé en ce que la cellule hôte est une cellule de levure.

**Revendications pour l'Etat contractant suivant : ES**

1. Procédé pour préparer un dérivé de l'hirudine, de séquence d'aminoacides

```
    0    1                                              10
   Leu-Thr-Tyr-Thr-Asp-Cys-Thr-Glu-Ser-Gly-Gln-Asn-Leu-Cys-
                                20
   Leu-Cys-Glu-Gly-Ser-Asn-Val-Cys-Gly-Gln-Gly-Asn-Lys-Cys-
                    30                                  40
   Ile-Leu-Gly-Ser-Asp-Gly-Glu-Lys-Asn-Gln-Cys-Val-Thr-Gly-
                                50
   Glu-Gly-Thr-Pro-Lys-Pro-Gln-Ser-His-Asn-Asp-Gly-Asp-Phe-
                    60
   Glu-Glu-Ile-Pro-Glu-Glu-Tyr-Leu-Gln,
```

caractérisé en ce qu'on exprime un ADN, qui code pour ce polypeptide, dans une cellule hôte.

2. Procédé selon la revendication 1, caractérisé en ce que la cellule hôte est une cellule de levure.

# Fig. 1

# Fig. 2

**a**

pαfHir17

2μ, Leu2, BamHI⁻/EcoRI⁻, 2μ, α, HIR, KpnI, pBR, BamHI, NcoI

**b**

pαfHir18

2μ, BamHI, NcoI, Leu2, HIR, KpnI, 2μ, α, pBR, EcoRI⁻/BamHI⁻